Europäisches Patentamt

European Patent Office

Office européen des brevets

(11) Publication number: **0 674 843 A1**

# EUROPEAN PATENT APPLICATION

(21) Application number: 95104751.3

(51) Int. Cl.6: **A23K 1/165**, C12N 9/42

(22) Date of filing: 30.03.95

(30) Priority: **31.03.94 JP 83769/94**

(43) Date of publication of application:
**04.10.95 Bulletin 95/40**

(84) Designated Contracting States:
**DE ES FR IT**

(71) Applicant: **MEIJI SEIKA KAISHA LTD.**
**4-16 Kyobashi 2-chome**
**Chuo-ku**
**Tokyo (JP)**

(72) Inventor: **Shimizu, Takao, c/o Meiji Seika**
**Kaisha, Ltd.**
**Pharmaceutical Research Lab.,**
**760, Morooka-cho**
**Kohoku-ku,**
**Yokohama-shi,**
**Kanagawa 222 (JP)**
Inventor: **Kiriya, Susumu, c/o Meiji Seika**
**Kaisha, Ltd.**
**Pharmaceutical Research Lab.,**
**760, Morooka-cho**
**Kohoku-ku,**
**Yokohama-shi,**
**Kanagawa 222 (JP)**
Inventor: **Okada, Tadaaki, c/o Meiji Seika**
**Kaisha, Ltd.**
**Pharmaceutical Research Lab.,**
**760, Morooka-cho**
**Kohoku-ku,**
**Yokohama-shi,**
**Kanagawa 222 (JP)**
Inventor: **Kajii, Kenzo, c/o Meiji Seika Kaisha,**
**Ltd.**

**Pharma-Technology Res. Lab.,**
**788, Kayama**
**Odawara-shi,**
**Kanagawa 250 (JP)**
Inventor: **Uotani, Kazumichi, c/o Meiji Seika**
**Kaisha, Ltd.**
**4-16, Kyobashi 2-chome**
**Chuo-ku,**
**Tokyo (JP)**
Inventor: **Kinoshita, Motoharu, c/o Meiji Seika**
**Kaisha, Ltd.**
**4-16, Kyobashi 2-chome**
**Chuo-ku,**
**Tokyo (JP)**
Inventor: **Kawasaki, Yoshikuni, c/o Meiji Seika**
**Kaisha, Ltd.**
**4-16, Kyobashi 2-chome**
**Chuo-ku,**
**Tokyo (JP)**
Inventor: **Kasuya, Kumiko, c/o Meiji Seika**
**Kaisha, Ltd.**
**4-16, Kyobashi 2-chome**
**Chuo-ku,**
**Tokyo (JP)**
Inventor: **Hashimoto, Kiyoshi, c/o Meiji Seika**
**Kaisha, Ltd.**
**4-16, Kyobashi 2-chome**
**Chuo-ku,**
**Tokyo (JP)**

(74) Representative: **Wilhelms, Rolf E., Dr.**
**WILHELMS, KILIAN & PARTNER**
**Patentanwälte**
**Eduard-Schmid-Strasse 2**
**D-81541 München (DE)**

(54) **A feed for domestic animals containing cellulase.**

(57) The present invention is directed to a feed a containing cellulase obtainable by a microorganism belonging to the genus *Acremonium* and a method for improving the growth and the feed conversion rate of animals which comprises feeding the animals with a feed containing cellulase obtainable by a microorganism belonging to the genus *Acremonium*.

Compared with the cellulase conventionally used as a feed additive, cellulase obatinable by a microorganism belonging to the genus *Acremonium* exerts a high decomposability on crystalline cellulose and a potent capability of saccharifying cellulose into glucose. Thus the present invention aims at improving the feeding efficiency of animals by adding this cellulase to an animal feed to thereby promote the effective utilization of the nutritive components of the feed.

FIELD OF THE INVENTION

This invention relates to a feed for domestic animals containing cellulase obtainable by a publicly known microorganism belonging to the genus *Acremonium* (JP-B-60-43954; the term "JP-B" as used herein means an "examined Japanese patent publication") and a method for improving the growth and the feed conversion rate of domestic animals which comprises feeding the animals with the above-mentioned feed.

BACKGROUND OF THE INVENTION

It has been attempted to add cellulase to feeds so as to effectively utilize the nutritive components of assorted feeds and use has been mainly made of cellulases originating in microorganisms belonging to the genus *Trichoderma* or Aspergillus therefor.

However, the cellulases originating in microorganisms belonging to the genus *Trichoderma* or Aspergillus, which have been employed hitherto, have some disadvantages of exhibiting only an unsatisfactory ability to decompose natural cellulose and failing to completely decompose cellulose into glucose and thus forming cellobiose or celooligosaccharides in a large amount. When such a cellulase is added to a feed, therefore, it cannot satisfactorily improve the utilization efficiency of the nutritive components of the feed, though it can decompose cellulose.

In order to solve the above-mentioned problem, the present inventors have conducted extensive studies to find out a strain capable of producing a cellulase which has an excellent decomposability on crystalline cellulose and a potent capability of saccharifying cellulose into glucose. As a result, they have found out that a cellulase produced by a microorganism belonging to the genus *Acremonium* (hereinafter, sometimes referred to simply as *Acremonium* cellulase or ACC) is an enzyme which is highly excellent in the capability of saccharification.

This cellulase exerts a strong decomposability on crystalline cellulose and has a much stronger β-glucosidase activity than those of the conventionally known cellulases such the one produced by *Trichoderma reesei*. Therefore, the addition of this *Acremonium* cellulase to a livestock feed makes it possible to accelerate the effective utilization of the nutritive components of the feed and improve the body weight gain and the feed conversion rate of domestic animals.

SUMMARY OF THE INVENTION

Accordingly, the present invention relates to a feed containing cellulase obtainable by a microorganism belonging to the genus *Acremonium* and a method for improving the growth and the feed conversion rate of domestic animals which comprises feeding the animals with the above-mentioned feed.

DETAILED DESCRIPTION OF THE INVENTION

As described above, the cellulase produced by a microorganism belonging to the genus *Acremonium* has such a strong decomposability on crystalline cellulose that it can decompose cellulose in a feed almost completely into glucose.

The *Acremonium* cellulase can be produced in accordance with, for example, the method described in JP-B-60-43954 by using *Acremonium cellulolyticus TN* (FERM BP-685) which has been deposited with Fermentation Research Institute, Agency of Industrial Science & Technology, Japan.

In the production process thereof, the microorganism is incubated and then the cells are removed from the culture medium by a solid/liquid separation procedure such as centrifugation. Then the supernatant thus obtained is further concentrated by ultrafiltration, etc. if required, and the moisture is eliminated from the concentrate by spray drying, etc. Thus a powdery preparation can be obtained. In the step of the spray drying, lactose, starch, or partially decomposed products of starch may be added, either alone or in the form of a mixture thereof, to the supernatant. Thus the process yield can be elevated and/or the physical properties (for example, solubility, stability) of the obtained powdery preparation can be improved.

To add to an animal feed, *Acremonium* cellulase may be formulated into an arbitrary form without restriction. For example, the above-mentioned powdery preparation may be used as such. Alternatively, it may be formulated into a preparation together with a filler (for example, wheat flour) by which the mixing can be facilitated. Other examples of the filler usable herein include corn meal, soybean flour, starch, partially decomposed products of starch, glucose, lactose, rice bran, wheat bran and mixtures thereof.

The addition level of *Acremonium* cellulase to the feed varies depending on the kinds of animals fed and feed selected. Usually, it may be used at a ratio of from 50 to 70 mg/kg body weight/day, preferably from 100 to 120 mg/kg body weight/day. In the case of a test with the use of Wistar rats as shown in the following Example, the concentration of *Acremonium* cellulase in a feed is preferably adjusted to at least 0.05 % by weight. Neither the timing nor the period of the addition is not restricted. Namely, *Acremonium* cellulase can be used throughout the feeding period. It is considered that pig and chicken are similar to rat in the growth and digestion ratio [Chikusan Shikenjo Kenkyu Hokoku (Research Report of National Institute of Livestock Industry), 14, 63 - 67, July, 1972; Nippon Chikusan Gakkaiho (Report of Japan Society of Livestock Industry), 52 (6), 459 - 466, June, 1981; etc.]. Thus the above-mentioned effects might be achieved even at an addition level lower than the one employed in the above-mentioned Example by taking the feeding period into consideration.

The present invention is applicable mainly to domestic animals such as cattle, horse, pig, chicken, goat, sheep and rabbit.

To examine the safety of *Acremonium* cellulase, an acute toxicity test was performed by suspending 1,000 mg/kg of *Acremonium* cellulase in water and orally administering the suspension to 3 male Wistar rat by force. As a result, these animals all survived without showing any clinical change. Thus it can be concluded that this *Acremonium* cellulase is a highly safe substance.

"The present invention is now illustrated in greater detail with reference to Production Examples and Examples, but it should be understood that the present invention is not to be construed as being limited thereto. All the percentages are by weight unless otherwise indicated."

PRODUCTION EXAMPLE 1

20 ml of a medium (pH 4.0) containing 4 % of cellulose, 1 % of bactpeptone, 0.6 % of potassium nitrate, 0.2 % of urea, 0.16 % of potassium chloride, 0.12 % of magnesium sulfate, 1.2 % of monopotassium phosphate, 0.001 % of zinc sulfate, 0.001 % of manganese sulfate and 0.001 % of copper sulfate was introduced into a 200 ml Erlenmeyer flask, sterilized in a conventional manner and inoculated with *Acremonium cellulolyticus* (FERM BP-685) followed by the incubation under aeration at 30 °C for 6 days. After the completion of the incubation, the culture medium was centrifuged to thereby give a supernatant. This supernatant had an avicelase activity of 3.1 U/ml, a CMCase activity of 26.3 U/ml and a $\beta$-glucosidase activity of 13.8 U/ml.

PRODUCTION EXAMPLE 2

20 ml of a medium (pH 4.0) containing 4 % of lactose, 1 % of bactpeptone, 0.6 % of potassium nitrate, 0.2 % of urea, 0.16 % of potassium chloride, 0.12 % of magnesium sulfate, 1.2 % of monopotassium phosphate, 0.001 % of zinc sulfate, 0.001 % of manganese sulfate and 0.001 % of copper sulfate was introduced into a 200 ml Erlenmeyer flask, sterilized in a conventional manner and inoculated with a variant of *Trichoderma reesei* followed by the incubation under aeration at 28 °C for 7 days. After the completion of the incubation, the culture medium was centrifuged to thereby give a supernatant. This supernatant had an avicelase activity of 10.3 U/ml, a CMCase activity of 96.2 U/ml and a cellobiase activity of 36.2 U/ml.

EXAMPLE 1

Use was made of pig feeds (prepared from corn, roasted wheat, wheat flour, wheat bran, soybean oil cake, etc., composed of 14.6 % of crude protein, 3.6 % of crude fat, 3.0 % of crude fiber, 3.8 % of crude ash, 0.74 % of calcium, 0.65 % of phosphorus, 11.4 % of digestible crude protein and 76.1 % of total digestible nutrients, and having 335 cal/100 g of digestible energy) containing *Acremonium* cellulase or *Trichoderma* cellulase added thereto. 5 g of each feed was weighed into a 250 ml baffle and 20 ml of deionized water and 1 drop of hydrochloric acid were added thereto (pH value: about 5.8). After shaking at 42 °C for 7 hours at 200 rpm, the mixture was centrifuged at 15,000 rpm for 10 minutes. The supernatant was filtered through a membrane filter (0.45 μm) and the filtrate was employed as a sample solution. This sample solution was analyzed in the amount of reducing sugars liberated (determined by the DNS method), the amount of glucose (determined by using a glucose biosensor) and the total nitrogen amount (determined by the semimicro-Kjeldahl method).

The results are summarized in Table 1. Compared with the *Trichoderma* cellulase, the *Acremonium* cellulase samples showed 2.2 times (in the 0.2 % lot) and 2.5 times (in the 2.0 % lot) as much reducing sugar contents and 2.5 times and 4.4 times as much glucose contents. The total nitrogen contents of the *Acremonium* cellulase samples of both lots were 1.3 times as much as those of the *Trichoderma* cellulase samples.

Based on these results, it has been found out that *Acremonium* cellulase further promotes the decomposition of cellulose in a feed, elevates the amounts of reducing sugars and glucose liberated and thus increases the amount of the total nitrogen liberated, compared with *Trichoderma* cellulase which has been employed conventionally. Thus it is suggested that the addition of *Acremonium* cellulase to a feed would accelerate the effective utilization of not only saccharides but also nitrogen sources, from among the nutritive components of the feed, compared with the conventional *Trichoderma* cellulase.

TABLE 1

| Comparison of *Acremonium* cellulase with *Trichoderma* cellulase in artificial digestion test of pig feed | | | | |
|---|---|---|---|---|
| Addition Level (%) | Cellulase-Producing Strain | Liberated | | |
| | | Reducing Sugar (mg/ml) | Glucose (mg/ml) | Total Nitrogen (ppm) |
| 0 | - | 8.5 | 4.5 | 71 |
| 0.1 | *Acremonium* | 19.3 | 9.8 | 92.7 |
| | *Trichoderma* | 11.6 | 5.3 | 80.5 |
| 0.2 | *Acremonium* | 27.6 | 14.7 | 112 |
| | *Trichoderma* | 12.6 | 6.0 | 87 |
| 2.0 | *Acremonium* | 60.9 | 38.4 | 242 |
| | *Trichoderma* | 24.3 | 8.7 | 186 |

EXAMPLE 2

24 male Wistar rats aged 4 weeks were classified into 3 groups each having 8 animals. A control (no addition) feed was given to the group 1, a feed containing 0.05 % of *Acremonium* cellulase was given to the group 2, and a feed containing 0.2 % of *Acremonium* cellulase was given to the group 3. A test feed for pigs was employed as the starting feed. The administration and the observation were each continued for 3 weeks. During this period, the body weight and the feed intake were measured and the body weight gain and the feed conversion rate were calculated.

Table 2 shows the average body weight gain, the average feed conversion rate and the improvements therein.

TABLE 2

| Body weight gain, feed conversion rate and improvement therein in rat feeding test | | | |
|---|---|---|---|
| Test Group No. | ACC Addition Level (%) | Body Weight Gain (g) in 21 Days (Improvement: %) | Feed Conversion Rate in 21 Days (Improvement: %) |
| 1 | 0 | 105.3 | 3.518 |
| 2 | 0.05 | 116.4* (10.5) | 3.324* (5.5) |
| 3 | 0.2 | 114.8 ( 9.0) | 3.297**(6.3) |

*: $P < 0.05$.
**: $P < 0.01$.

As Table 2 clearly shows, the test groups 2 and 3 showed each good body weight gain, though no dose-dependency was observed therein. A significant difference ($P < 0.05$) was observed between the 0.05 % group, and the control group and the improvement was about 10 % in this case. On the other hand, the feed conversion rate was improved depending on the dose. Namely, significant differences ($P < 0.01$, $P < 0.05$) were observed between the control group and the 0.2 % group, and between the control group and the 0.05 % group. The improvements were 6.3 % and 5.5 %, respectively. These results indicate that the addition of *Acremonium* cellulase to a feed would promote the body weight gain and improve the feed conversion rate.

The improvement was calculated in accordance with the following formula.

$$\text{Improvement (\%)} = \frac{\text{Difference from control group}}{\text{Data of control group}} \times 100$$

On the other hand, the feed conversion rate was calculated in accordance with the following formula.

$$\text{Feed conversion rate} = \frac{\text{Feed intake (g) in 21 days}}{\text{Body weight gain (g)}}$$

In a similar test with the use of *Trichoderma* cellulase, the body weight gain was elevated by about 2 % but no improvement was observed in feed conversion rate. Namely, both of these factors showed no significant difference.

The present invention provides a feed containing cellulase obtainable by a microorganism belonging to the genus *Acremonium* (JP-B-60-43954). By feeding animals with this feed, the growth and the feed conversion rate of the animals can be improved.

## Claims

1. A feed containing cellulase obtainable by a microorganism belonging to the genus *Acremonium*.

2. The feed as set forth in Claim 1 or 2, wherein said feed comprises an ordinary feed for domestic animals and a cellulase obtainable by a microorganism belonging to the genus Aremonium.

3. The feed as set forth in Claim 1 or 2, wherein said microorganism belonging to the genus Aremonium is *Acremonium cellylolyticus TN* (FERM BP-685).

4. A method for improving the growth and the feed conversion rate of animals which comprises feeding the animals with a feed containing cellulase obtainable by a microorganism belonging to the genus *Acremonium*.

5. The use of a composition, which comprises an ordinary feed for domestic animals and a cellulase obtainable by a microorganism belonging to the genus Acremonium, for preparing a composition fo rimproving the growth and the feed conversion rate of animals.

6

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP-A-0 188 050 (AGENCY OF INDUSTRIAL SCIENCE & TECHNOLOGY MINISTRY ) * page 16, last paragraph * * claims 1-7 * | 1-5 | A23K1/165 C12N9/42 |
| X | DATABASE WPI Week 9222 Derwent Publications Ltd., London, GB; AN 92-180059 & JP-A-04 117 244 (AGENCY OF IND SCI & TECHNOLOGY) , 17 April 1992 * abstract * | 1,2 | |
| X | JOURNAL OF JAPANESE SOCIETY OF GRASSLAND SCIENCE, vol. 39, no. 3, 1993 JP, pages 317-325, T.B. BAYORBOR ET AL. 'Effects of Acremonium cellulase and lactic acid bacteria inoculant on the fermentation quality and digestibility of Guineagrass' * page 317, Synopsis * * page 318, paragraph 3 - paragraph 4 * * page 319, last paragraph - page 323, paragraph 4 * | 1,2 | TECHNICAL FIELDS SEARCHED (Int.Cl.6) A23K C12N |
| X | US-A-4 562 150 (TAKASHI YAMANOBE ET AL.) * column 9, line 3 - line 10 * * claims 1,2 * | 1,2 | |

The present search report has been drawn up for all claims

| Place of search | Date of completion of the search | Examiner |
|---|---|---|
| THE HAGUE | 16 June 1995 | Dekeirel, M |